# EUROPEAN PATENT SPECIFICATION

(11) **EP 4 281 443 B1**
(45) Date of publication and mention of the grant of the patent: **19.02.2025**
(21) Application number: 22700995.8
(22) Date of filing: 21.01.2022
(51) Int. Cl.: C07D 311/40, C07D 311/92, C07C 67/08, C07C 69/84, C07D 311/04

(54) **PHARMACEUTICAL PRODUCTS BASED ON CANNABINOID ACID ESTERS**
PHARMAZEUTISCHE PRODUKTE AUF BASIS VON CANNABINOIDSÄUREESTERN
PRODUITS PHARMACEUTIQUES À BASE D'ESTERS D'ACIDE CANNABINOÏDE

(30) Priority: 22.01.2021 GR 20210100041
(43) Date of publication of application: 29.11.2023
(73) Proprietor: EKATI ALCHEMY LAB, S.L., 08108 Moia (Barcelona) (ES); ETHNIKO KAI KAPODISTRIAKO PANEPISTIMIO ATHINON, 10679 Athens (GR); Magiatis, Prokopios, 18902 Salamina (GR); Melliou, Eleni, 18902 Salamina (GR); Dadiotis, Evangelos, 19010 Kalyvia (GR)
(72) Inventor: MITSIS, Vangelis, 08108 MOIA (BARCELONA) (ES); MAGIATIS, Prokopios, 18902 SALAMINA (GR); MELLIOU, Eleni, 18902 SALAMINA (GR); DADIOTIS, Evangelos, 19010 KALYVIA (GR)
(74) Representative: Araujo, Daniel
(86) International application number: PCT/EP2022/051402
(87) International publication number: WO 2022/157338

(56) References cited:
- EP-A1- 2 913 321
- WO-A1-2019/033164
- CARDILLO G ET AL: "Synthesis of d,l-cannabichromene, franklinone and other natural chromenes", TETRAHEDRON, ELSEVIER SIENCE PUBLISHERS, AMSTERDAM, NL, vol. 24, no. 13, 1 January 1968 (1968-01-01), pages 4825 - 4831, XP026645860, ISSN: 0040-4020, [retrieved on 19680101], DOI: 10.1016/S0040-4020(01)98678-8
- CHAN T H ET AL: "A Biomimetic Synthesis of A'-Tetrahydrocannabinol", TETRAHEDRON LETTERS, 1 January 1982 (1982-01-01), pages 2935 - 2938, XP055844229, Retrieved from the Internet <URL:https://www.sciencedirect.com/science/article/pii/S0040403900874985> [retrieved on 20210923]

## Description

### Field of the invention

This application is related to a new method of preparing cannabinoid acid esters directly from selected plant material of the genus *Cannabis sp,* preferably *Cannabis sativa,* by simultaneously extracting the cannabinoid acids and synthesizing their respective esters during the extraction process. In addition, the application concerns pharmaceutical preparations containing cannabinoid acid esters, directly obtained from the above method and their use for the production of therapeutic preparations. The application concerns the field of medicine and pharmacy.

### Background of the invention

The *Cannabis sp* plant, preferably *Cannabis sativa,* contains terpenophenolic compounds called cannabinoids. Cannabinoids refer to the compounds that interact and bind to the cannabinoid receptors CB1 and CB2. Compounds that bind to cannabinoid receptors are endocannabinoids, phytocannabinoids and synthetic cannabinoids. Cannabinoid acid refers to the carboxylic acid substituted cannabinoid. Cannabinoid acid ester refers to the ester form of cannabinoid acids, where the ester substituent is attached to the carboxylic group of cannabinoid acid.

In the plant are mainly present cannabinoid acids with general formula I, II, III, IV with R1 = H.

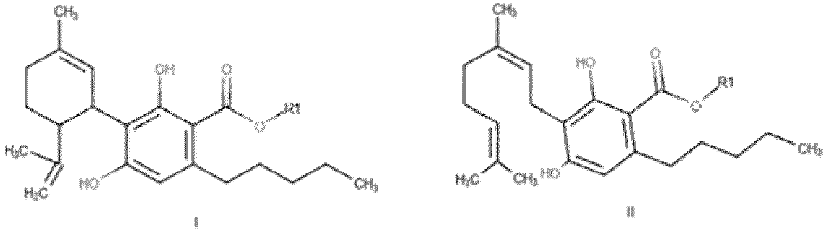

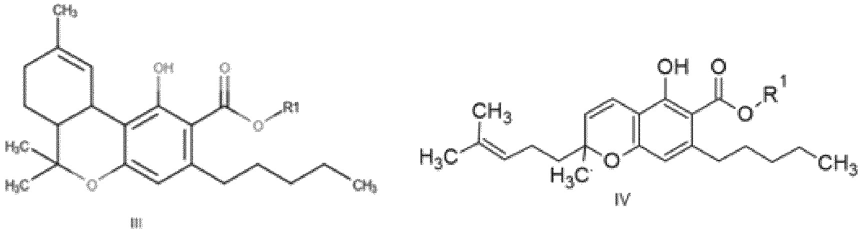

Cannabidiolic acid (CBDA) (I), cannabigerolic acid (CBGA) (II) and cannabichromenic acid (CBCA) (III) are the main non-psychotropic phytocannabinoids of *Cannabis sativa* and tetrahydrocannabinolic acid (THCA) is the main psychoactive substance of C. *sativa,* where with selected plant material and with a suitable way of extraction, the extract can contain up to 80% of these substances. Cannabinoid acids are considered precursors of the neutral cannabinoids, such as cannabigerol (CBG) which can be produced by decarboxylation of cannabigerolic acid. Due to the instability of cannabinoids, their chemical form has been studied for the first time in the past with the methyl ester analysis of each cannabinoid (Mechoulam & Gaoni; Tetrahedron. 1965 May;21(5):1223-9).

The glandular hairs of the inflorescences of the plant Cannabis sp. preferably, Cannabis sativa, contain the highest percentage of phytocannabinoids. Cultivar varieties mainly, contain CBDA and / or CBGA and / or CBCA and / or THCA.

CBDA, CBGA, CBCA and THCA have been evaluated for their anti-cancer activity and their results are part of US2020030282 A1 Patent Application. CBDA has been shown to inhibit cell migration and enhance the expression of cyclooxygenase-2 in breast cancer cells MDA-MB-231 (Hirao-Suzuki et al.; J Toxicol Sci. 2020; 45 (4): 227-236, Hirao- Suzuki et al., Nat Prod Commun. 2017 May; 12 (5): 759-761. Takeda et al.; Toxicol Lett. 2012 Nov 15; 214 (3): 314-9).

However, the activity of acids is higher in *in vitro* experiments than in *in vivo* experiments, due to the reduced lipophilicity of cannabinoid acids, which leads to limited penetration into biological membranes (Smeriglio et al .; Fitoterapia. 2018 June; 127: 101-8 ). The inventors of their experiments know that by creating esters, the molecules become more lipophilic with increased penetrating capability, which may contribute to increasing their activity. The general types of cannabinoid esters are:

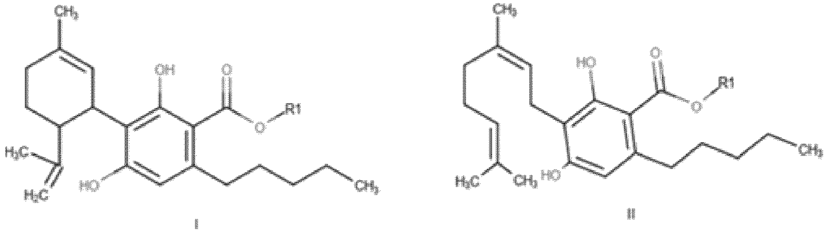

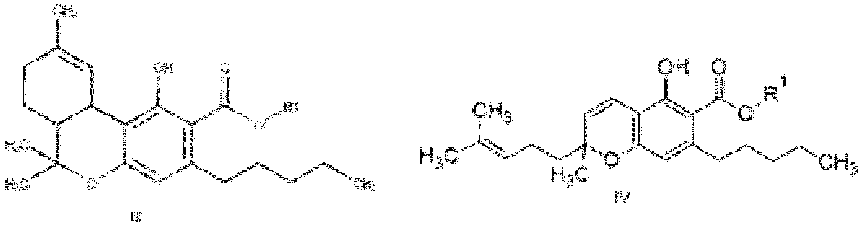

R1 consists of a straight or branched alkyl group, alkenyl group or alkynyl group having from 3 to 5 carbons.

For cannabinoid acid methyl esters there are published synthesis methods, for example in WO2019033164 A1 and EP2913321 A1. However, the published synthesis methods require chromatographic purification of the substances before and after the esterification, which makes the process quite complicated, while increases the cost of the final products. CARDILLO G ET AL: "Synthesis of d,l-cannabichromene, franklinone and other natural chromenes", TETRAHEDROM, ELSEVIER SIENCE PUBLISHERS, AMSTERDAM, NL, discloses ethyl esters of type (II) and (IV) wherein R1 is an alkyl group having two carbons. Chan T H ET AL: "A Biomimetic Synthesis of A'-Tetrahydrocannabinol", Tetrahedron Letters, discloses a methyl ester of type (III) wherein R1 is an alkyl group having two carbons. CBDA esters have been studied in various pharmacological targets e.g., based on the application of WO2020186010 A1, but never so far for their anti-cancer properties, while the esters of CBGA, CBCA and THCA have never been pharmacologically evaluated. In the present invention the above esters were evaluated and for the first time significant anti-cancer activity was found.

The synthesis of the esters described in the present invention involves the simultaneous extraction and esterification of the substances from the C. *sativa* plant, as the solvent used for the extraction simultaneously leads to the esterification. This process is original and at the same time more economical and less time consuming than the synthesis methods mentioned in the literature, without requiring chromatographic purification of the substances. The novelty is in particular the fact that the esterification reagents, 4-dimethylaminopyridine (DMAP) or pyridine or triethylamine or any other tertiary nitrogenous base and 1-Ethyl-3-(3-dimethylaminopropyl)carbodiimide (EDC) or N,N'-Diisopropylcarbodiimide or any other conjugating agent with hydrophilic by-products, are not inhibited by the other components of the plant material and at the same time the resulting product can be easily separated from the other components of the plant material without the need for chromatography. In contrast, the use of standard N,N'-Dicyclohexylcarbodiimide (DCC) coupling reagent is not appropriate as it does not produce hydrophilic by-products and therefore requires the use of chromatography to purify cannabinoids.

Therefore, an easier and faster way of extraction and synthesis of cannabinoid esters can lead to a wider pharmacological investigation of these substances and consequently to an easier production of pharmaceutical compositions consisting of cannabinoid acid esters.

### Summary of the invention

According to one embodiment, the present invention relates to a process for the production of esters from the corresponding cannabinoid acids during the simultaneous extraction of the cannabinoid acids from the C. sativa plant.

In another embodiment, the invention relates to cannabinoid acid esters of general formula I or II or III or IV, readily obtained from the method mentioned above.

In a further embodiment, the invention relates to cannabinoid acid esters of general formula I or II or III or IV for use in the treatment of cancer.

In yet another embodiment, the invention relates to cannabinoid acid esters of general formula I or II or III or IV for use in the treatment of psoriasis.

In another embodiment, the invention relates to cannabinoid acid esters of general formula I or II or III or IV for use in the treatment of degenerative diseases of the central nervous system.

In another embodiment, the invention relates to cannabinoid acid esters of general formula I or II or III or IV for use in the treatment of chronic pain.

In yet another embodiment, the invention relates to pharmaceutical compositions comprising cannabinoid acid esters of general formula I or II or III or IV.

### Description of the drawings

**Figure 1****.** Determination of the cytotoxic activity of CBDA and CBGA methyl esters in SK-BR-3 cancer cells in 48 hours.
**Figure 2****.** Determination of the cytotoxic effect of CBDA and CBGA methyl esters on SK-BR-3 cancer cells in 72 hours.
**Figure 3****.** Comparison of the cytotoxic activity of CBDA methyl ester in SK-BR-3 cancer cells compared to the cytotoxic activity in normal MCF10A cells.

### Detailed description of the embodiments

The present invention and its various embodiments refer to a novel process for the production of cannabinoid acid esters of general formula I or II or III or IV, wherein R1 consists of a straight or branched alkyl group, alkenyl group or alkynyl group having from 3 to 5 carbons, during the simultaneous extraction of cannabinoid acids derived from the plant *Cannabis sp.,* Preferably *Cannabis sativa,* into esters of cannabinoid acids with general formula I or II or III or IV prepared by this particular method, and pharmaceutical compositions containing esters of cannabinoid acids of general formula I or II or III or IV for use in medical treatment.

More specifically, in one embodiment, the present invention relates to a process for the production of esters from the corresponding cannabinoid acids during the simultaneous extraction of the cannabinoid acids from the *C*. *sativa* plant.

In another embodiment, the invention relates to cannabinoid acid esters of general formula II or III or IV, wherein R1 consists of a straight or branched alkyl group, alkenyl group or alkynyl group having from 3 to 5 carbons, readily obtained from the method mentioned above.

In a further embodiment, the invention relates to cannabinoid acid esters of general formula II or III or IV for use in the treatment of cancer, preferably breast cancer.

In yet another embodiment, the invention relates to cannabinoid acid esters of general formula II or III or IV for use in the treatment of psoriasis.

In another embodiment, the invention relates to cannabinoid acid esters of general formula II or III or IV for use in the treatment of degenerative diseases of the central nervous system.

In another embodiment, the invention relates to cannabinoid acid esters of general formula II or III or IV for use in the treatment of chronic pain.

In yet another embodiment, the invention relates to pharmaceutical compositions comprising cannabinoid acid esters of general formula II or III or IV, as obtained by the method mentioned above.

In another embodiment, the invention relates to pharmaceutical compositions comprising cannabinoid acid esters of general formula II or III or IV in the form of an oral solution, solution for injection, transdermal solution, suppository or tablet.

In yet another embodiment, the invention relates to pharmaceutical compositions comprising cannabinoid acid esters of general formula II or III or IV and one or more pharmaceutically acceptable excipients.

According to some embodiments the pharmaceutically acceptable excipient is an aqueous solution or carrier. In some embodiments the aqueous solution is a buffer at normal pH or near normal, such as Phosphate Buffer Saline (PBS). In some embodiments the pharmaceutically acceptable excipient may be an emulsifier, a buffering agent, a pH adjusting agent, a tonic modifier, a preservative, an antioxidant, a stabilizer or a combination of the above.

Although the present invention is described with reference to specific embodiments thereof, it will be appreciated by one skilled in the field, that various variations may be made and equivalent alternatives may be used without departing from the scope of the invention. The same is true of the following examples which are used to illustrate in practice and clearly ways of carrying out the invention and not to limit it.

### Principle of the method for the preparation of cannabinoid acid esters

The first part of the invention presents the synthesis of cannabinoid acid esters with general formula I or II or III or IV from the corresponding cannabinoid acids during the simultaneous extraction of cannabinoid acids from selected plant material of *C*. *sativa.*

The plant material used for the extraction includes the inflorescences and the glandular hairs of the plant C. sativa. The synthesis of each ester is carried out in parallel with the extraction, using a different alcoholic extraction solvent. The extraction medium is also a reagent for the synthesis of esters and the R1 substituent is determined by the solvent to be used for the extraction. The solvent may be any straight or branched alcohol having carbon numbers from 1 to 5. Preferably the solvent is a primary or secondary alcohol, in liquid phase and at room temperature. The extraction of the plant material is carried out in an ultrasonic bath. This method of extraction is very efficient and at the same time favours the formation of esters. The same procedure can be performed without an ultrasonic bath, with stirring and with or without heating of the solvent but with reduced extraction efficiency and consequently with reduced reaction efficiency. In this case, despite the lower efficiency, the process may be preferred due to lower cost and large-scale applicability. The extraction is carried out in the presence of the reagents 1-Ethyl-3-(3-dimethylaminopropyl)carbodiimide (EDC) and 4-dimethylaminopyridine (DMAP), which are used to synthesize the esters.

The reagents (EDC and DMAP) added during the extraction to the ester composition give hydrophilic by-products, which are water-soluble and remain insoluble in organic solvents.

Purification of the extract from the by-products of the reaction occurring in parallel with the extraction is performed by changing the pH of the extract and then by rinsing with water, without the need of chromatographic purification to obtain the final product.

### Example 1 - Method for simultaneous extraction and production of cannabinoid acid esters from the Cannabis sativa plant.

The simultaneous extraction and production of cannabinoid acid esters from the *Cannabis sativa* plant involves the following steps:
a. The glandular hairs of the female inflorescences of the *C. sativa* plant variety are obtained, which contain high concentrations of the substances with general formula I or II or III or IV with R1 = H. An analysis is made of the content of plant material in these substances prior to the procedure. The plant material is immersed in an alcoholic solvent in a ratio of 1 part dry plant material: 10 - 100 parts solvent. This is followed by the addition of DMAP in a ratio of 0.1 - 1 mol DMAP: 1 mol of cannabinoid acids and the addition of EDC in a ratio of 1 - 2 mol of EDC: 1 mol of cannabinoid acids. The mixture is placed in an ultrasonic bath for 15 - 45 minutes and the cannabinoid acids are extracted from the plant and at the same time are converted to the corresponding esters.
b. The alcoholic solvent containing the esters of the cannabinoid acids is obtained by filtration to remove the plant material.
c. To the solution is added dilute HCl solution (0.1-1 M) or another strong inorganic acid, which makes the reaction by-products water-soluble. The HCl solution is mixed with the alcoholic solution, and a mixture of solvents is formed which has more hydrophilic properties. The esters are then obtained by liquid-liquid extraction with an organic solvent (e.g. hexane) which forms a biphasic system with the alcohol-water mixture.
d. The organic solvent is rinsed with an alkaline solution and water and evaporated to dryness and the esters are obtained with more than 90% purity in the respective substances depending on the plant material and on the alcoholic solvent that used

### Example 1a:

To 1.5 g of C. sativa plant material with a CBGA content of 30% by dry weight 150 ml of methanol are added. 1.25 mmol DMAP and 2.5 mmol EDC are added to the mixture. The mixture is placed in an ultrasonic bath for 25 minutes. The mixture is then filtered to remove the plant material and the methanol is obtained. To the solution are added 50 ml of 0.5M HCl solution. Then, 50 ml of hexane are added forming a two-phase solvent system and the hexane phase (organic) containing the CBGA-Me is obtained. The organic phase is rinsed with saturated sodium bicarbonate solution and water. The organic phase is evaporated to dryness to obtain 441.2 mg of CBGA-Me. ¹ H-NMR (400 MHz, CDCl₃): 6.28 (1H, s), 5.28 (1H, t, 7.0 Hz), 5.07 (1H, t, 6.6 Hz), 3.92 (3H, s), 3.42 (2H, d, 7.0Hz), 2.09 (2H, t, 6.6 Hz), 2.09 (2H, q, 6.5 Hz), 2.81 (2H, t, 7.6 Hz), 2.07 (2H, m), 1.81 (3H, s), 1.67 ( 3H, s), 1.59 (3H, s), 1.35 (2H, m), 1.35 (2H, m), 0.90 (3H, t, 6.9 Hz).

### Example 1b:

To 1.5 g of C. sativa plant material with a CBGA content of 30% by dry weight 150 ml of ethanol is added. 1.25 mmol DMAP and 2.5 mmol EDC are added to the mixture. The mixture is placed in an ultrasonic bath for 30 minutes. The mixture is then filtered to remove the plant material and the ethanol is taken up. To the solution is added 50 ml of 0.5M HCl solution. Then, 50 ml of hexane are added and a biphasic solvent system is formed and the hexane phase containing the CBGA-Et is obtained. The organic phase is rinsed with saturated sodium bicarbonate solution and water. The organic phase is evaporated to dryness to obtain 458.3 mg CBGA-Et. ¹H-NMR (400 MHz, CDCl₃): 6.28 (1H, s), 5.28 (1H, t, 7.0 Hz), 5.07 (1H, t, 6.6 Hz), 4.39 (2H, q, 7.1 Hz), 3.41 (2H) , d, 7.0Hz), 2.09 (2H, t, 6.6 Hz), 2.09 (2H, q, 6.5 Hz), 2.82 (2H, t, 7.6 Hz), 2.07 (2H, m), 1.81 (3H, s) , 1.67 (3H, s), 1.59 (3H, s), 1.40 (3H, t, 7.1 Hz), 1.35 (2H, m), 1.35 (2H, m), 0.90 (3H, t, 6.9 Hz).

### Example 1c:

To 1.5 g of C. sativa plant material with a CBDA content of 30% by dry weight 150 ml of methanol is added. 1.25 mmol DMAP and 2.5 mmol EDC are added to the mixture. The mixture is placed in an ultrasonic bath for 25 minutes. The mixture is then filtered to remove the plant material and the methanol is taken up. To the solution is added 50 ml of 0.5M HCl solution. 'Then, 50 ml of hexane are added and a biphasic solvent system is formed and the hexane phase containing the CBDA-Me is obtained. The organic phase is rinsed with saturated sodium bicarbonate solution and water. The organic phase is evaporated to dryness to obtain 444.6 mg CBDA-Me. ¹H-NMR (400 MHz, CDCl₃): 6.23 (1H, s), 5.57 (1H, s), 4.53 (1H, m), 4.23 (1H, m), 4.11 (1H, m), 3.92 (3H, s) ), 2.93 (1H, m), 2.83 (1H, m), 2.40 (m), 2.21 (1H, m), 2.10 (1H, m), 1.86 (m), 1.81 (3H, s), 1.72 (3H , s), 1.57 (2H, m), 1.35 (8H, m), 0.89 (3H, t, 6.9 Hz).

### Example 1d:

To 1.5 g of C. sativa plant material with a CBDA content of 30% by dry weight ml of ethanol are added. 1.25 mmol DMAP and 2.5 mmol EDC are added to the mixture. The mixture is placed in an ultrasonic bath for 25 minutes. The mixture is then filtered to remove the plant material and the ethanol is taken up. To the solution is added 50 ml of 0.5M HCl solution. 'Then, 50 ml of hexane are added to form a biphasic solvent system and the hexane phase containing the CBDA-Et is obtained. The organic phase is rinsed with saturated sodium bicarbonate solution and water. The organic phase is evaporated to dryness to obtain 459.7 mg CBDA-Et. ¹H-NMR (400 MHz, CDCl₃): 6.28 (1H, s), 5.28 (1H, t, 7.0 Hz), 5.07 (1H, t, 6.6 Hz), 4.39 (2H, q, 7.1 Hz), 3.41 (2H) , d, 7.0Hz), 2.09 (2H, t, 6.6 Hz), 2.09 (2H, q, 6.5 Hz), 2.82 (2H, t, 7.6 Hz), 2.07 (2H, m), 1.81 (3H, s) , 1.67 (3H, s), 1.59 (3H, s), 1.40 (3H, t, 7.1 Hz), 1.35 (2H, m), 1.35 (2H, m), 0.90 (3H, t, 6.9 Hz).

### Example 1e:

To 1.5 g of C. sativa plant material with a CBGA content of 30% by dry weight 150 ml of isopropanol is added. 1.25 mmol DMAP and 2.5 mmol EDC are added to the mixture. The mixture is placed in an ultrasonic bath for 40 minutes. The mixture is then filtered to remove plant material and isopropanol is obtained. To the solution is added 50 ml of 0.5M HCl solution. Then, 50 ml of hexane are added and a two-phase solvent system is formed and the hexane phase containing the CBGA-iPro is obtained. The organic phase is rinsed with saturated sodium bicarbonate solution and water. The organic phase is evaporated to dryness to obtain 451.2 mg CBGA-iPro. ¹H-NMR (400 MHz, CDCl₃): 6.24 (1H, s), 5.33 (1H, m), 5.28 (1H, t, 7.0 Hz), 5.07 (1H, t, 6.6 Hz), 3.92 (3H, s) , 3.43 (2H, d, 7.0Hz), 2.09 (2H, t, 6.6 Hz), 2.09 (2H, q, 6.5 Hz), 2.83 (2H, t, 7.6 Hz), 2.07 (2H, m), 1.81 ( 3H, s), 1.67 (3H, s), 1.59 (3H, s), 1.35 (2H, m), 1.41 (3H, s), 1.39 (3H, s), 1.35 (2H, m), 0.90 (3H , t, 6.9 Hz).

### Example 1f:

To 1.5 g of C. sativa plant material with a CBCA content of 6% by dry weight 150 ml of methanol is added. To the mixture are added 0.25 mmol DMAP and 0.5 mmol EDC. The mixture is placed in an ultrasonic bath for 25 minutes. The mixture is then filtered to remove the plant material and the methanol is taken up. To the solution is added 50 ml of 0.5M HCl solution. 'Then, 50 ml of hexane are added and a biphasic solvent system is formed and the hexane phase containing the CBCA-Me is obtained. The organic phase is rinsed with saturated sodium bicarbonate solution and water. The organic phase is evaporated to dryness to obtain 84 mg CBCA-Me. ¹H-NMR (400 MHz, CDCl₃): 6.74 (1H, d, 10.1 Hz), 6.23 (1H, s), 5.48 (1H, d, 10.1 Hz), 5.21 (1H, m), 3.93 (3H, s) , 2.81 (2H, t, 6.9Hz), 1.95 (2H, m), 1.67 (3H, s), 1.59 (3H, s), 1.55 (2H, m), 1.41 (3H, s), 0.9 (3H, m), 1.55 (2H, m), 1.32 (4H, m).

### Example 1g:

To 1.5 g of C. sativa plant material with a THCA content of 30% by dry weight 150 ml of ethanol is added. 1.25 mmol DMAP and 2.5 mmol EDC are added to the mixture. The mixture is placed in an ultrasonic bath for 30 minutes. The mixture is then filtered to remove the plant material and the ethanol is taken up. To the solution is added 50 ml of 0.5M HCl solution. 'Then, 50 ml of hexane are added and a two-phase solvent system is formed and the hexane phase containing the THCA-Et is obtained. The organic phase is rinsed with saturated sodium bicarbonate solution and water. The organic phase is evaporated to dryness to obtain 458 mg THCA-Et. ¹H-NMR (400 MHz, CDCl₃): 6.41 (1H, brs), 6.22 (1H, s), 4.41 (2H, 7.1 Hz), 3.23 (1H, dm, 10.9 Hz), 2.89 (1H, m), 2.74 (1H, m), 2.18 (2H, m), 1.93 (1H, m), 1.68 (3H, s), 1.67 (m), 1.57 (2H, m), 1.44 (3H, s), 1.42 (3H, t, 7.1 Hz), 1.35 (4H, m), 1.11 (3H, s), 0.90 (3H, t, 6.9 Hz)

### Example 2

### Example 2a - Preparation of oral solution with cannabinoid acid esters

The CBGA methyl ester from Example 1 (1000 mg) is dissolved in olive oil (20 grams) and placed in a vial with dropper for use as an oral solution in drops.

### Example 2b - Preparation of soft capsules with cannabinoid acid esters

The final product of the concentrate from any of Examples 1a-1d is mixed with olive oil in a ratio of 1:10 by weight and incorporated into a soft capsule.

### Example 2c - Preparation of hard capsules with cannabinoid acid esters

The final product of the concentrate from any of Examples 1a-1d is mixed with microcrystalline cellulose in a ratio of 1:20 by weight and can be used to make hard capsules.

### Example 3 - Study of the pharmaceutical properties of cannabinoid acid esters

The cytotoxic activity of CBDA methyl esters, CBCA CBGA and THCA was studied by the in vitro MTT colorimetric process (Mosmann, T. et al. J. Immunol. Methods. 1983; 65: 55-63). This method, which is widely used to measure cellular metabolic activity as an indicator of cell viability, division and cytotoxicity, is based on the reduction of a yellow salt of tetrazole ((3-(4,5-dimethylthiazol-2-yl) -2). 5-diphenyltetrazolium bromide or MTT) in violet formazan crystals from metabolically active cells.

Briefly for this cytotoxicity test, the SK-BR-3 cancer cell line (human breast cancer cell line), the MCF-7 cancer cell line (human breast cancer cell line), and the A2058 cancer cell line (series) were used. melanoma) and the cancer line SKMEL28 (melanoma line). All cell lines were tested at 20% v / v O2, in the presence of 10% FBS after 48h or 72h of incubation

The study of the cytotoxic activity of CBGA, CBCA and THCA CBDA methyl esters by the MTT assay showed that substances in concentrations less than 40 µM can lead to cell death in 50% of SK- cancer cells. BR-3 MCF7, A2058 and SKMEL28 and therefore these substances and any medicinal products derived from them can be used to treat breast cancer and melanoma.

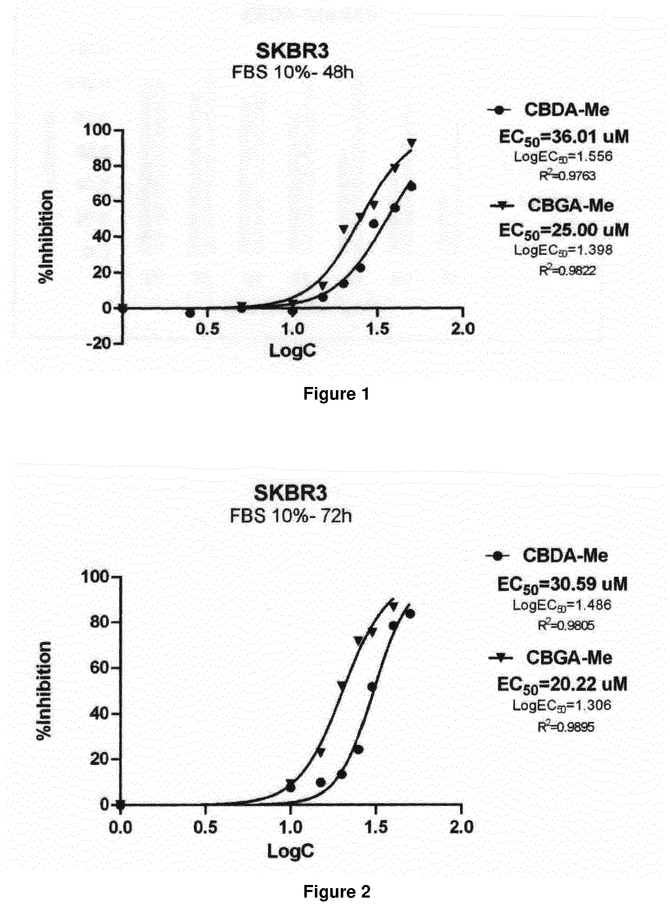

In the SKBR3 series, the greatest activity was shown by CBGA methyl ester, which for example had much better activity than CBDA methyl ester at 48 h and 72 h (Figures 1 and 2). Specifically, in cancer cell line SKBR3 CBGA-Me has EC50 = 20.2 µM while CBD-Me has EC50 = 30.6 µM, CBCA-Me has EC50 = 35 µM and THCA-Me has EC50 = 36 µM.

In comparison, the action of esters is much stronger than the known corresponding decarboxylated products. For example, CBGA methyl ester is almost twice as active as CBG at 72 h. Specifically, in cancer cell line SKBR3 oCBGA-Me has EC50 = 20.2 µM while CBG has EC50 = 38.8 µM

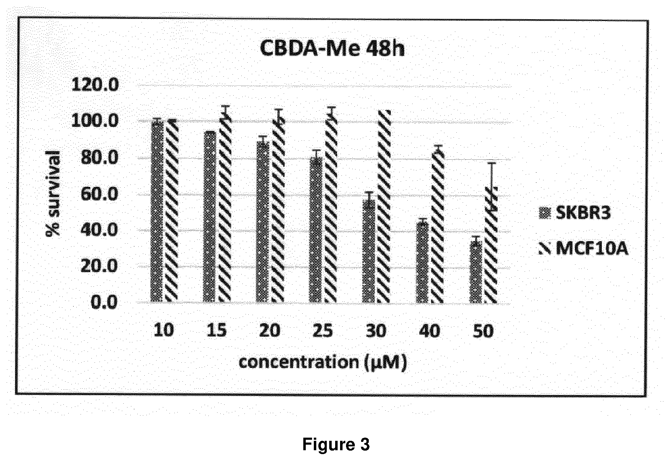

The esters also have a much stronger effect on SK-BR-3 cancer cells than normal MCF10A cells as shown in Figure 3 in the case of CBDA methyl ester.

In MCF7 cell line CBGA methyl ester showed EC50 activity = 18 µM.

Regarding the two melanoma cancer lines, CBGA methyl ester showed EC50 = 17 µM.

The improved activity and lipophilicity of esters, in relation to their carboxylated and decarboxylated analogues having a number of known actions such as protection against neurodegenerative diseases, which makes them potentially effective in all therapeutic applications of cannabinoids.

## Claims

1. A method for the preparation of cannabinoid acids having general formula (I, II, III, IV), 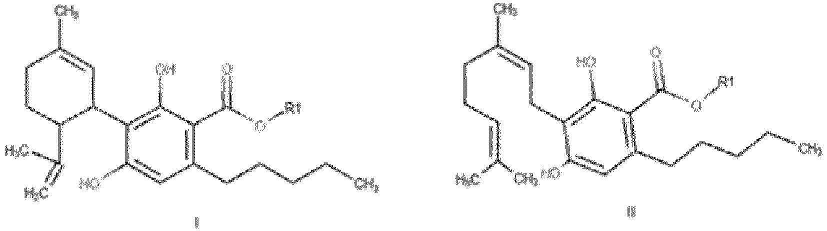 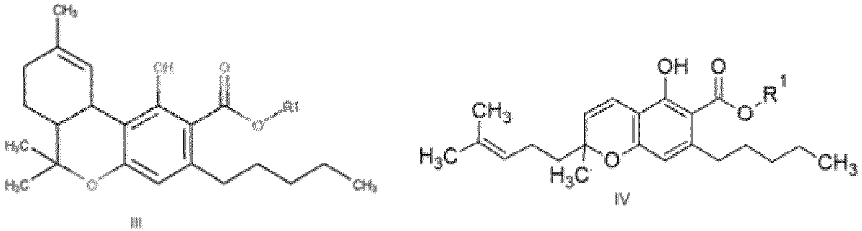 wherein
R1 = linear or branched alkyl group, alkenyl group or alkynyl group having from 1 to 5 carbons;
wherein the method includes the following steps:
a) immersing plant material of the Cannabis sp plant containing substances of formula I or II or III or IV with R1 = H in alcohol, straight or branched and with a number of carbon atoms from 1 to 5, in a ratio of dry plant material: alcohol from 1:10 to 1: 100;
b) adding 4-dimethylaminopyridine (DMAP) to the mixture, in a ratio of mol DMAP: mol of cannabinoid acids from 1:10 to 1: 1;
c) adding 1-ethyl-3-(3-dimethylaminopropyl)carbodiimide (EDC) to the mixture, in a ratio of 2 mol EDC: 1 mol of cannabinoid acids;
d) placing the mixture in an ultrasonic bath for 15 to 45 minutes;
e) filtering the mixture to remove plant material;
f) adding an acid solution to the alcoholic solution obtained from step e;
g) performing a liquid-liquid extraction with an organic solvent immiscible with the alcohol-water mixture obtained from step f;
h) washing the organic solvent with an aqueous alkaline solution and water; and
i) evaporating the organic solvent to dryness to obtain the cannabinoid acid esters of formula I or II or III or IV, with R1 = straight or branched alkyl group, alkenyl group or alkynyl group having from 1 to 5 carbons.

2. Cannabinoid acid esters having general formula (II) or (III) or (IV) individually or in any combination thereof, 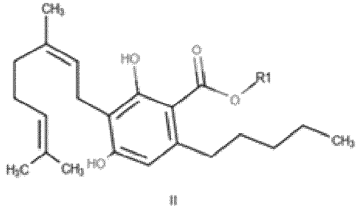 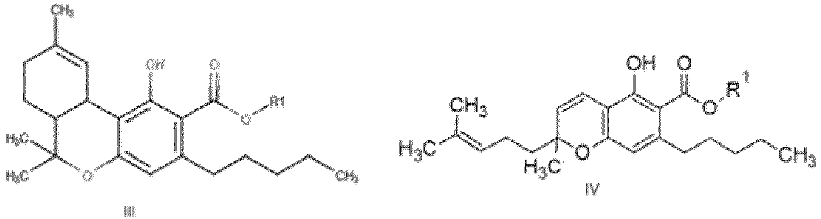 wherein
R1 = linear or branched alkyl group, alkenyl group or alkynyl group having from 3 to 5 carbons;

3. A pharmaceutical composition comprising the cannabinoid esters of claim 2 and a pharmaceutically acceptable excipient.

4. A pharmaceutical composition according to claim 3 for use in the treatment of cancer.

5. A pharmaceutical composition according to claim 3 for use in the treatment of breast cancer.

6. A pharmaceutical composition according to claim 3 for use in the treatment of skin cancer, preferably melanoma.

7. A pharmaceutical composition according to any one of claims 3-6 in the form of an oral solution.

8. A pharmaceutical composition according to any one of claims 3-6 in the form of a solution for injection.

9. A pharmaceutical composition according to any one of claims 3-6 in the form of a transdermal solution.

10. A pharmaceutical composition according to any one of claims 3-6 in the form of a tablet or suppository.

11. A pharmaceutical composition according to any one of claims 3-6 in the form of a soft or hard capsule.

## Patentansprüche

1. Verfahren zum Herstellen von Cannabinoidsäuren mit der allgemeinen Formel (I, II, III, IV), 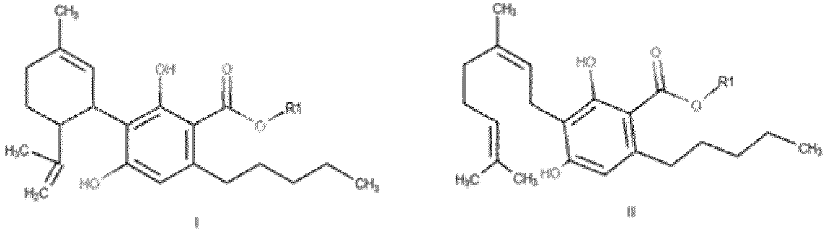 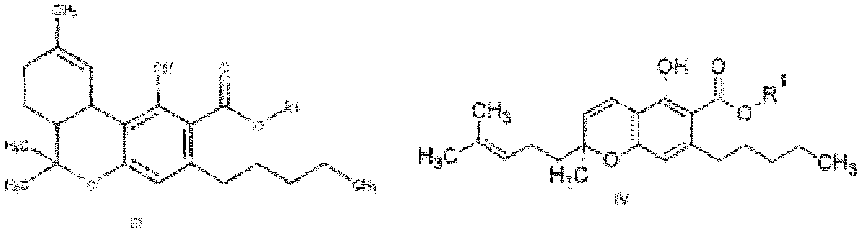 wobei
R1 = geradkettige oder verzweigte Alkylgruppe, Alkenylgruppe oder Alkinylgruppe mit 1 bis 5 Kohlenstoffatomen;
wobei das Verfahren die folgenden Schritte beinhaltet:
a) Eintauchen von Pflanzenmaterial der Pflanze *Cannabis* sp., das Substanzen der Formel I oder II oder III oder IV mit R1 = H enthält, in Alkohol, geradkettig oder verzweigt und mit einer Anzahl von Kohlenstoffatomen von 1 bis 5, in einem Verhältnis von Trockenpflanzenmaterial:Alkohol von 1:10 bis 1:100;
b) Hinzufügen von 4-Dimethylaminopyridin (DMAP) zu dem Gemisch in einem Verhältnis von Mol DMAP:Mol Cannabinoidsäuren von 1:10 bis 1:1;
c) Hinzufügen von 1-Ethyl-3-(3-dimethylaminopropyl)carbodiimid (EDC) zu dem Gemisch in einem Verhältnis von 2 Mol EDC:1 Mol Cannabinoidsäuren;
d) Platzieren des Gemischs in einem Ultraschallbad für 15 bis 45 Minuten;
e) Filtern des Gemischs, um Pflanzenmaterial zu entfernen;
f) Hinzufügen einer Säurelösung zu der alkoholischen Lösung, die in Schritt e erhalten wird;
g) Durchführen einer Flüssig-Flüssig-Extraktion mit einem organischen Lösungsmittel, das mit dem Alkohol-Wasser-Gemisch, das in Schritt f erhalten wird, nicht mischbar ist;
h) Waschen des organischen Lösungsmittels mit einer wässrigen alkalischen Lösung und Wasser; und
i) Verdampfen des organischen Lösungsmittels bis zur Trockne, um die Cannabinoidsäureester der Formel I oder II oder III oder IV zu erhalten, wobei R1 = geradkettige oder verzweigte Alkylgruppe, Alkenylgruppe oder Alkinylgruppe mit 1 bis 5 Kohlenstoffatomen.

2. Cannabinoidsäureester der allgemeinen Formel (II) oder (III) oder (IV) einzeln oder in einer beliebigen Kombination davon, 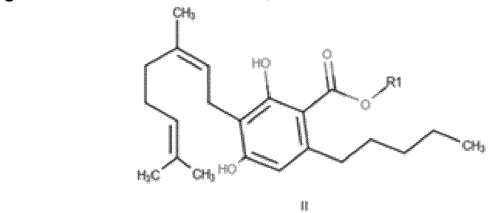 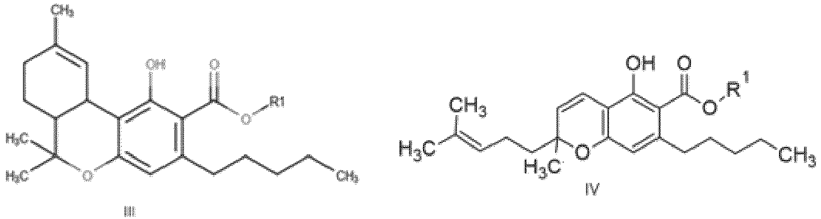 wobei
R1 = geradkettige oder verzweigte Alkylgruppe, Alkenylgruppe oder Alkinylgruppe mit 3 bis 5 Kohlenstoffatomen.

3. Pharmazeutische Zusammensetzung, umfassend die Cannabinoidester nach Anspruch 2 und einen pharmazeutisch verträglichen Hilfsstoff.

4. Pharmazeutische Zusammensetzung nach Anspruch 3 zur Verwendung bei der Behandlung von Krebs.

5. Pharmazeutische Zusammensetzung nach Anspruch 3 zur Verwendung bei der Behandlung von Brustkrebs.

6. Pharmazeutische Zusammensetzung nach Anspruch 3 zur Verwendung bei der Behandlung von Hautkrebs, vorzugsweise Melanom.

7. Pharmazeutische Zusammensetzung nach einem der Ansprüche 3-6 in Form einer oralen Lösung.

8. Pharmazeutische Zusammensetzung nach einem der Ansprüche 3-6 in Form einer Injektionslösung.

9. Pharmazeutische Zusammensetzung nach einem der Ansprüche 3-6 in Form einer transdermalen Lösung.

10. Pharmazeutische Zusammensetzung nach einem der Ansprüche 3-6 in Form einer Tablette oder eines Zäpfchens.

11. Pharmazeutische Zusammensetzung nach einem der Ansprüche 3-6 in Form einer Weich- oder Hartkapsel.

## Revendications

1. Procédé pour la préparation d'acides cannabinoïdes répondant à la formule générale (I, II, III, IV), 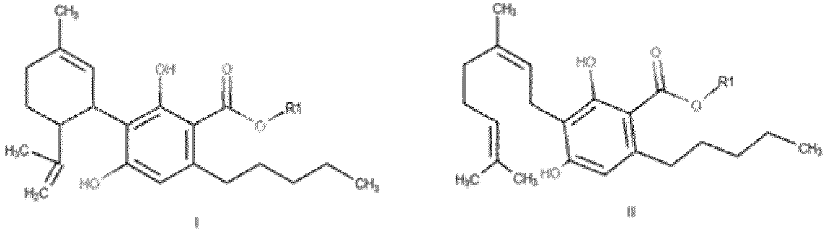 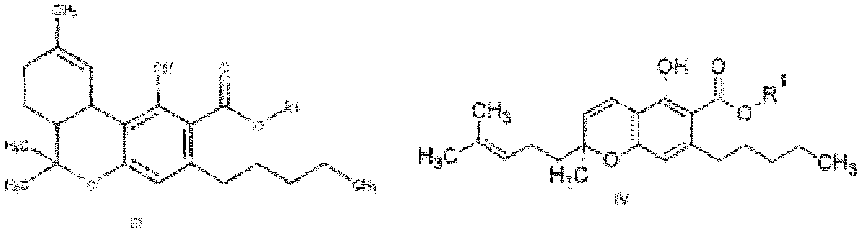 où
R1 = un groupe alkyle, groupe alcényle ou groupe alcynyle linéaire ou ramifié ayant de 1 à 5 carbones ;
le procédé comprenant les étapes suivantes :
a) l'immersion de matériel végétal de la plante Cannabis sp contenant des substances de formule I ou Il ou III ou IV où R1 = H dans de l'alcool, droit ou ramifié et dont le nombre d'atomes de carbone va de 1 à 5, en un rapport matière végétale sèche:alcool allant de 1:10 à 1:100 ;
b) l'ajout de 4-diméthylaminopyridine (DMAP) au mélange, en un rapport nombre de moles de DMAP:nombre de moles d'acides cannabinoïdes allant de 1:10 à 1:1 ;
c) l'ajout de 1-éthyl-3-(3-diméthylaminopropyl)carbodiimide (EDC) au mélange, en un rapport de 2 moles d'EDC:1 mole d'acides cannabinoïdes ;
d) le placement du mélange dans un bain à ultrasons pendant 15 à 45 minutes ;
e) la filtration du mélange pour éliminer le matériel végétal ;
f) l'ajout d'une solution acide à la solution alcoolique obtenue à partir de l'étape e ;
g) la mise en œuvre d'une extraction liquide-liquide avec un solvant organique non miscible avec le mélange alcool-eau obtenu à partir de l'étape f ;
h) le lavage du solvant organique avec une solution aqueuse alcaline et de l'eau ; et
i) l'évaporation du solvant organique jusqu'à sec pour obtenir les esters d'acides cannabinoïdes de formule I ou II ou III ou IV, où R1 = un groupe alkyle, groupe alcényle ou groupe alcynyle droit ou ramifié ayant de 1 à 5 carbones.

2. Esters d'acides cannabinoïdes répondant à la formule générale (II) ou (III) ou (IV) individuellement ou en une quelconque combinaison de ceux-ci, 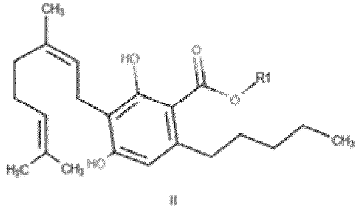 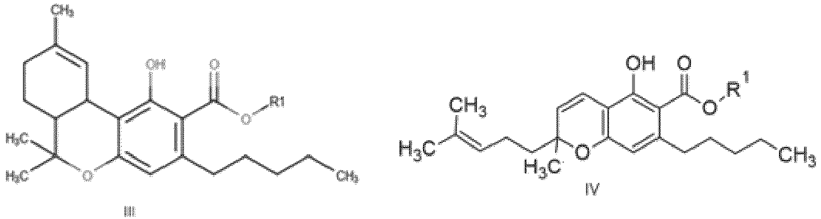 où
R1 = un groupe alkyle, groupe alcényle ou groupe alcynyle linéaire ou ramifié ayant de 3 à 5 carbones.

3. Composition pharmaceutique comprenant les esters de cannabinoïdes selon la revendication 2 et un excipient pharmaceutiquement acceptable.

4. Composition pharmaceutique selon la revendication 3 destinée à être utilisée dans le traitement d'un cancer.

5. Composition pharmaceutique selon la revendication 3 destinée à être utilisée dans le traitement d'un cancer du sein.

6. Composition pharmaceutique selon la revendication 3 destinée à être utilisée dans le traitement d'un cancer de la peau, de préférence d'un mélanome.

7. Composition pharmaceutique selon l'une quelconque des revendications 3 à 6 sous la forme d'une solution orale.

8. Composition pharmaceutique selon l'une quelconque des revendications 3 à 6 sous la forme d'une solution pour injection.

9. Composition pharmaceutique selon l'une quelconque des revendications 3 à 6 sous la forme d'une solution transdermique.

10. Composition pharmaceutique selon l'une quelconque des revendications 3 à 6 sous la forme d'un comprimé ou d'un suppositoire.

11. Composition pharmaceutique selon l'une quelconque des revendications 3 à 6 sous la forme d'une capsule molle ou dure.
